# EUROPÄISCHE PATENTSCHRIFT

(11) **EP 0 904 630 B1**
(45) Veröffentlichungstag und Bekanntmachung des Hinweises auf die Patenterteilung: **09.07.2008**
(21) Anmeldenummer: 98919196.0
(22) Anmeldetag: 31.03.1998
(51) Int. Cl.: H02K 15/14, H02K 11/00

(54) **VERFAHREN ZUR HERSTELLUNG EINES MIKROMOTORS**
METHOD FOR PRODUCING A MICROMOTOR
PROCEDE DE FABRICATION D'UN MICROMOTEUR

(30) Priorität: 02.04.1997 US 832040
(43) Veröffentlichungstag der Anmeldung: 31.03.1999
(73) Patentinhaber: Abiomed Europe GmbH, 52074 Aachen (DE)
(72) Erfinder: SIESS, Thorsten, D-52146 Würselen (DE)
(74) Vertreter: Selting, Günther
(86) Internationale Anmeldenummer: PCT/EP1998/001867
(87) Internationale Veröffentlichungsnummer: WO 1998/044619

(56) Entgegenhaltungen:
- EP-A- 0 361 775
- DE-A- 2 805 659
- US-A- 4 482 829
- US-A- 4 534 420
- US-A- 5 199 171
- PATENT ABSTRACTS OF JAPAN vol. 018, no. 155 (E-1524), 15. März 1994 & JP 05 328655 A (MITSUBISHI ELECTRIC CORP), 10. Dezember 1993

## Beschreibung

Die Erfindung betrifft ein Verfahren zur Herstellung eines Mikromotors, insbesondere für eine Blutpumpe, die intrakardial oder intravasal betrieben werden kann.

Bekannt sind intravasale Blutpumpen, die in den Körper eingeführt werden, indem ein Blutgefäß des vaskulären Systems punktiert und anschließend die Blutpumpe durch das Blutgefäß hindurch an den gewünschten Einsatzort im Innern des Körpers vorgeschoben wird. Der maximale Durchmesser solcher Komponenten, die in Blutgefäße eingeführt werden, ist begrenzt, da die Pumpe auf dem Weg von ihrem Einführungsort bis zum Einsatzort durch das Blutgefäßsystem vorgeschoben werden muß. Außerdem darf die Blutpumpe nur geringe axiale Abmessungen haben, so daß ihr Vorschub durch Biegungen des Gefäßsystems nicht behindert wird. Im Falle, daß eine Blutpumpe zur Linksherzunterstützung bis zum Herzen vorgeschoben wird, darf der Außendurchmesser 8,0 mm nicht überschreiten und die starre Länge der Pumpe darf 4 cm nicht überschreiten, damit die Pumpe durch den Aortenbogen in das Herz geschoben werden kann.

Aus EP 0 157 871 B1 und EP 0' 397 668 sind intravasale Herzpumpen bekannt, bei denen der Pumpenteil und der Motorteil getrennt ausgeführt und durch eine flexible Welle miteinander verbunden sind. Während der Pumpenteil in den Körper eingeführt wird, verbleibt der Motorteil außerhalb des Körpers.

In WO94/09835 (Jarvik) ist eine Pumpvorrichtung für die Herzunterstützung beschrieben, bei der mindestens eine Pumpe, bei der der Pumpenteil und der Motorteil starr miteinander verbunden sind, mit dem Pumpenteil in einen Ventrikel des geöffneten Herzens eingeführt wird, während der Motorteil außerhalb des Herzens verbleibt.

Es besteht ein Bedarf an intrakardialen oder intravasalen Pumpen, also Pumpen, die vollständig im Herzen oder in einem Blutgefäß angeordnet und betrieben werden können und bei denen der Motorteil und der Pumpenteil eine starre Einheit bilden. Dies setzt jedoch voraus, daß jeder dieser beiden Teile extrem kleinformatig und mit hoher Präzision hergestellt werden kann.

In WO97/37696 (nicht vorveröffentlicht) ist eine intravasale Blutpumpe beschrieben, bei der der Antriebsteil und der Pumpenteil eine starre Einheit bilden, die mit einem Katheter verbunden ist. Die Blutpumpe hat so geringe Abmessungen, daß sie durch ein Blutgefäß hindurch an den Einsatzort geschoben werden kann oder auch im Blutgefäß betrieben werden kann. Bei der intravasalen Blutpumpe haben der Pumpenteil und der Antriebsteil im wesentlichen gleichen Durchmesser, der nicht größer ist als etwa 5 - 7 mm. Die Pumpe kann zusätzlich mit einem flexiblen Schlauch verlängert sein, der ihre effektive Länge vergrößert.

Bei einer im Körper zu betreibenden Blutpumpe ist eine hohe Präzision des Antriebsteils und des Pumpenteils erforderlich. Insbesondere muß sichergestellt sein, daß das Flügelrad der Rotationspumpe sehr genau im Pumpengehäuse zentriert ist, und daß das Pumpengehäuse in bezug auf das Motorgehäuse ebenfalls sehr präzise angeordnet ist. Wenn diese Bedingungen nicht genau eingehalten werden, können infolge von Scherkräften Blutschädigungen und Thrombenbildung auftreten. Andererseits ist zu berücksichtigen, daß derartige Blutpumpen in der Regel als Einmal-Artikel benutzt werden und daher kostengünstig herstellbar sein müssen.

Ein Verfahren zur Herstellung eines Miniaturmotors ist bekannt aus DE 28 05 659 A1. Bei diesem Verfahren wird in eine Spritzgussform ein Dorn eingebracht, an dem Statorkomponenten des herzustellenden Stators befestigt werden. Anschließend wird der Formhohlraum mit einem Polymermaterial ausgespritzt, wobei die Statorkomponenten, zu denen auch Spulen gehören, in das Polymermaterial eingebettet werden. Anschließend wird die Spritzgussform geöffnet und der Dorn wird herausgezogen. Anstelle des Dornes wird ein Rotor in den Stator eingeführt.

US 4 482 829 beschreibt ein Verfahren zur Herstellung eines bürstenlosen elektrischen Mikromotors. Dabei werden die Feldwicklungen eines Stators mit einem synthetischen Harzmaterial umspritzt. Die so entstandene Hülse wird in ein Statorgehäuse aus magnetischem Material eingesetzt. In dem Statorgehäuse ist ein abdichtendes Rotorgehäuse untergebracht, in dem ein Rotor in Lagern gelagert ist.

EP 0 361 775 beschreibt eine von einem Motor angetriebene Trommel für eine Waschmaschine, wobei ein Motorelement im Spritzgussverfahren durch Umspritzen ferromagnetischer Teile hergestellt wird. Beim Spritzgussprozess wird auch ein Sitz zur Aufnahme eines Lagers für die Rotorwelle erzeugt, jedoch nicht mit einem bereits eingesetzten Lager.

In US-A-5121021 ist ein Verfahren zur Herstellung des Stators einer dynamoelektrischen Maschine beschrieben, bei dem eine quergeteilte Spritzgussform verwendet wird, in deren Formhohlraum ein Dorn hineinragt. Der Dorn trägt Permanentmagnete, welche Statormagnete festhalten, welche als Statorkomponenten in das Kunststoffmaterial des Stators eingegossen werden. Weiterhin trägt der Dorn ein Gleitlager, welches ebenfalls mit dem KunststofFmaterial umspritzt wird. Das Gleitlager dient dazu, später die Welle des Motors zu lagern.

Das Umspritzen eines Lagers in einem Miniaturmotor ist ebenfalls beschrieben in US-A-5038460. Hierbei ist das zu umspritzende Lager ein Kugellager. Ein derartiger Miniaturmotor ist hauptsächlich für den Antrieb von Magnetplatten bestimmt. Er hat eine kurze Baulänge, jedoch ein Gehäuse mit relativ großer radialer Erstreckung.

JP 05328655 A beschreibt ebenfalls die Herstellung eines Motors im Spritzgussverfahren. Dabei werden Statorkomponenten wie Spulen und Magnetkerne auf einen Dorn aufgeschoben und in einen Formhohlraum eingebracht. Nach dem Einspritzen der Kunststoffmasse wird der Dorn aus dem entstandenen Motorgehäuse herausgezogen. Anstelle des Dornes wird ein Stator in das Gehäuse eingesetzt.

Der Erfindung liegt die Aufgabe zugrunde, ein Verfahren zur Herstellung eines für eine Blutpumpe verwendbaren Mikromotors anzugeben, das er erlaubt, einen Mikromotor mit geringen Kosten und hoher Präzision herzustellen.

Die Lösung dieser Aufgabe erfolgt erfindungsgemäß mit den im Patentanspruch 1 angegebenen Merkmalen.

Bei dem erfindungsgemäßen Verfahren wird der Stator des Mikromotors dadurch hergestellt, dass die Statorkomponenten auf einen Dorn aufgeschoben und zusammen mit dem Dorn in eine Spritzgussform eingebracht werden. In die Spritzgussform wird Polymermaterial injiziert, das die Statorkomponenten umschließt und anschließend das Motorgehäuse bildet. Auf diese Weise entsteht ein an dem Dorn zentrierter Stator, dessen Komponenten in ein Gehäuse eingebettet sind, das ebenfalls an dem Dorn zentriert ist. Der Dorn kann sehr präzise in der Spritzgußform positioniert werden. Die Statorkomponenten sind generell Spulen und Rückschlußweicheisenbleche für den magnetischen Rückschluß. Das erfindungsgemäße Herstellungsverfahren erlaubt es, rigorose Toleranzforderungen strikt zu erfüllen, indem der Stator nicht wie üblich von außen nach innen aufgebaut wird, sondern von innen nach außen. Die Zentrierung erfolgt an dem Dorn, auf den zuerst die Statorkomponenten und anschließend das Gehäuse aufgebracht wird. Nach dem Entfernen des Dornes kann anstelle des Dornes ein Motorrotor eingeführt werden, der dann sehr präzise positioniert ist.

Als Polymermaterial wird zweckmäßigerweise ein Material benutzt, das so niedrigviskos ist, daß es die einzelnen Bauteile umschließt und selbst in engste Spalte kriecht, so daß die einzelnen Bauteile nicht nur verkapselt, sondern fest miteinander verbunden werden. So werden die einzelnen Windungen und Statorbleche zu einer integralen Einheit verklebt. Für die Herstellung einer intravasalen Blutpumpe eignet sich insbesondere ein biokompatibles Zweikomponenten-Epoxydharz.

Bei dem erfindungsgemäßen Verfahren wird auf den Dorn ein Lager aufgeschoben, das zusammen mit dem Dorn in die Spritzgussform eingebracht wird.

Damit das Lager nicht mit der Elastomermasse ausgespritzt wird,kann es mit einem entfernbaren Material gefüllt sein, beispielsweise mit Wachs oder Silikonfett, das später ausblutet oder als Lagerschmierstoff wirkt.

Das erfindungsgemäße Verfahren eignet sich insbesondere für einen elektronisch kommutierten Synchronmotor, bei dem der Rotor mindestens einen Permanentmagneten enthält, während der Stator Spulenwicklungen enthält. Der aus Welle, Magnet und Dichtung bestehende Rotor bildet eine Einheit, die insgesamt in das Motorgehäuse eingeführt werden kann, wobei die Einführungsöffnung schließlich durch die zu der Einheit gehörende Dichtung verschlossen wird. Auch das Flügelrad der Pumpe kann fester Bestandteil der Rotoreinheit sein.

Vorzugsweise wird das Motorgehäuse mit einer dem Querschnitt des einzusetzenden Rotors entsprechenden Öffnung hergestellt, durch die der Rotor eingeschoben wird, auf dessen Welle ein die Öffnung verschließendes Lager sitzt. Dies ermöglicht es, den Mikromotor aus nur zwei Komponenten zusammenzusetzen. Der Mikromotor kann daher einfach und kostengünstig hergestellt werden und er eignet sich als Einmal-Artikel.

Im folgenden wird unter Bezugnahme auf die Zeichnungen ein Ausführungsbeispiel der Erfindung näher erläutert.

Es zeigen:
- Fig. 1: einen Längsschnitt durch den herzustellenden Mikromotor samt Pumpe,
- Fig. 2: einen Längsschnitt durch die Spritzgußform während der Herstellung des Motorgehäuses,
- Fig. 3: einen Längsschnitt durch die leere Spritzgußform und
- Fig.4: eine vergrößerte Darstellung der Einzelheit IV aus Fig. 2.

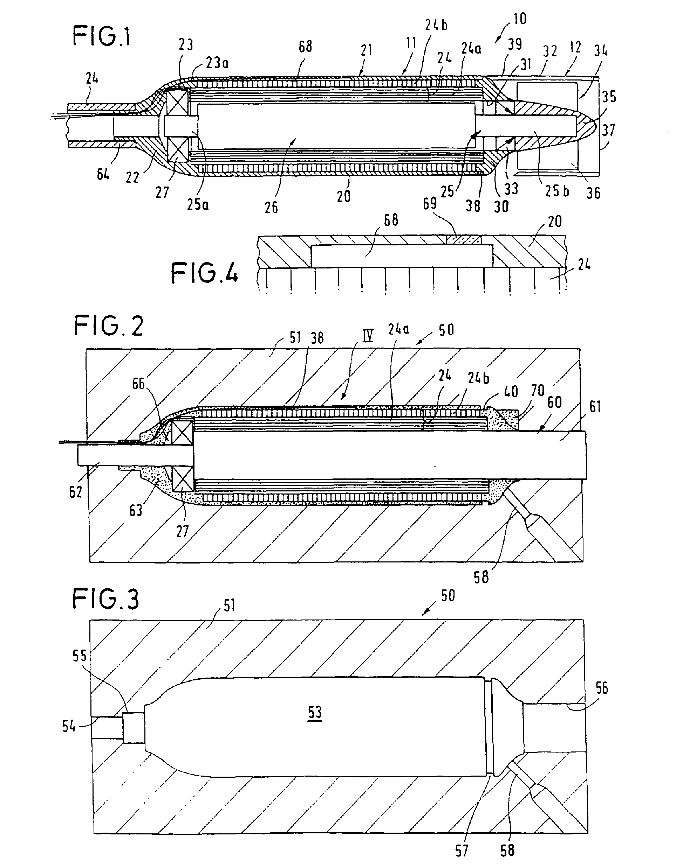

In Fig. 1 ist eine intravasale Blutpumpe 10 dargestellt, also eine Blutpumpe, die durch das Blutgefäßsystem eines Patienten geschoben werden kann, um bis in das Herz hinein vorzudringen. Der Außendurchmesser einer solchen Blutpumpe ist an keiner Stelle größer als 7 mm.

Die Pumpe 10 weist einen Antriebsteil 11 und einen damit starr verbundenen Pumpenteil 12 auf. Der Antriebsteil 11 enthält einen elektrischen Mikromotor 21 mit einem langgestreckten zylindrischen Gehäuse 20. An dem rückwärtigen Ende ist das Gehäuse 20 mit einer Stirnwand 22 verschlossen, an die sich ein flexibler Katheter 14 abdichtend anschließt. Durch den Katheter 14 verlaufen die elektrischen Kabel 23 zur Stromversorgung und zur Steuerung des Elektromotors 21, und außerdem weitere Kabel 23a, die mit Sensoren der Blutpumpe 10 verbunden sind.

Der Stator 24 des Motors weist in üblicher Weise zahlreiche umfangsmäßig verteilt angeordnete Wicklungen 24a sowie einen aus Blechen bestehenden magnetischen Rückschluß 24b in Längsrichtung auf. Er ist mit dem Motorgehäuse 20 umspritzt. Der Stator 24 umgibt den mit der Motorwelle 25 verbundenen Rotor 26, der aus in Wirkrichtung magnetisierten Permanentmagneten besteht. Die Motorwelle ist am rückwärtigen Ende mit einem Lager 27 im Motorgehäuse bzw. in der Stirnwand 22 gelagert. Die Welle erstreckt sich durch die gesamte Länge des Motorgehäuses 20 und ragt nach vorne aus diesem heraus.

Den vorderen Abschluß des Motorgehäuses bildet ein rohrförmiges stationäres Nabenteil 30, das integraler Bestandteil des Gehäuses 20 ist. Der Außendurchmesser des Nabenteils verjüngt sich zum vorderen Ende hin, wo sich ein Lager 33 zur Lagerung der Motorwelle 25 befindet. Dieses Lager ist zugleich als Wellendichtung ausgebildet.

Die Motorwelle 25 steht aus dem Nabenteil 30 nach vorne vor und trägt dort ein Flügelrad 34 mit einer auf dem Wellenende sitzenden Nabe 35 und davon abstehenden Flügeln 36 oder Pumpenschaufeln, die in bezug auf die Achse des Flügelrades 34 schräggestellt sind. Das Flügelrad 34 ist in einem zylindrischen Pumpengehäuse 32 enthalten, das durch drei umfangsmäßig verteilte Stege 39 mit einem Ring 38 verbunden ist, welcher auf dem Nabenteil 30 sitzt. Man erkennt, daß das Motorgehäuse 20 und das Pumpengehäuse 32 starr miteinander verbunden sind und gleiche Außendurchmesser haben, und daß der Durchmesser der Pumpe 10 an keiner Stelle größer ist als dieser Außendurchmesser.

Bei einer Rotation des Flügelrades 34 wird Blut durch die Ansaugöffnung 37 des Pumpengehäuses 32 angesaugt und in axialer Richtung im Pumpengehäuse 32 nach hinten getrieben. Durch den ringförmigen Spalt zwischen dem Pumpengehäuse 32 und dem Motorgehäuse 20 strömt das Blut am Nabenteil 30 entlang nach außen, um weiter an dem Motorgehäuse 20 entlangzuströmen. Hierdurch wird der Abtransport der im Antrieb erzeugten Wärme sichergestellt, ohne daß es zur Blutschädigung durch zu hohe Oberflächentemperaturen (über 41°C) auf dem Motorgehäuse 20 kommt.

Es ist auch möglich, den Pumpenteil 12 mit umgekehrter Förderrichtung zu betreiben, wobei das Blut an dem Motorgehäuse entlang angesaugt wird und aus der stirnseitigen Öffnung 37 axial austritt.

In die Umfangswand des Motorgehäuses 20 ist ein Drucksensor 68 eingebettet, der mit einer Leitung 23a in Verbindung steht. Diese Leitung 23a ist in dem Motorgehäuse 20 vergossen und sie führt durch die Stirnwand 22 hindurch in den Katheter 24. Am proximalen Katheterende sind die Leitung 23a und das Kabel 23 mit einem extrakorporalen Steuergerät verbindbar, das den Betrieb der Pumpe 10 steuert.

Die Herstellung des Mikromotors 21 erfolgt in einem simplen Spritzgußverfahren mit der in Fig. 3 dargestellten Spritzgußform. Diese Spritzgußform 50 besteht aus zwei Formhälften 51, die auseinanderfahrbar sind und im zusammengesetzten Zustand einen Formhohlraum 53 umschließen, dessen Kontur der Außenkontur des Gehäuses 20 entspricht.

Von dem Formhohlraum 53 erstreckt sich eine axiale Bohrung 54, die über einen Stufenabschnitt 55 erweiterten Durchmessers mit dem Formhohlraum 53 verbunden ist.

Am gegenüberliegenden Ende befindet sich eine axialBohrung 56, deren Durchmesser etwa so groß ist wie derjenige des Rotors 26. In dem Formhohlraum 53 ist ferner ein umlaufender Vorsprung 57 ausgebildet, um an dem Motorgehäuse eine Ringnut 40 zu erzeugen. In den Formhohlraum 53 führen Injektionskanäle 58 hinein, un Kunststoff in die Form zu injizieren.

Bei der Herstellung des Mikromotors wird der in Fig. 2 dargestellte Dorn 60 benutzt. Dieser besteht aus einem zylindrischen Stab 61, dessen Länge größer ist als diejenige des Stators 24. An das eine Ende des Stabes 61 schließt sich ein zylindrischer Ansatz 62 mit verringertem Durchmesser an, der exakt in die Bohrung 54 der Spritzgußform 50 hineinpaßt. Der zylindrische Stab 61 paßt genau in die axiale Bohrung 56, so daß eine genau zentrische Lage des Dornes 60 sicher erreicht werden kann.

Auf den Ansatz 62 wird das Lager 27 passend aufgeschoben. Auf den Stab 61 werden die Komponenten 24a,24b des Stators 24 aufgeschoben. Dann wird der Dorn 60 mit den Komponenten 24a,24b des Stators 24 und dem Lager 27 in das Formteil 51 eingelegt und schließlich wird die Spritzgußform durch Aufsetzen der zweiten Formhälfte verschlossen. Danach wird Kunststoff in den Formhohlraum 53 injiziert, wobei sich diejenigen Hohlräume, die nicht von auf dem Dorn 60 sitzenden Komponenten ausgefüllt werden, mit Polymermaterial 63 füllen, um so das Gehäuse 20 mit der Stirnwand 22 und dem Nabenteil 30 zu bilden. In dem Bereich des Stufenansatzes 55 entsteht eine Muffe 64 (Fig. 1), auf die der Katheter 24 aufgeschoben werden kann. Durch diese Muffe 64 hindurch verlaufen die Kabel 23 und die Leitung 23a. Auch das Nabenteil 30 wird aus dem injizierten Polymermaterial geformt, ebenso wie die Umfangswand des Gehäuses 20. In dem Nabenteil 30 entsteht eine Öffnung 31 (Fig. 1), die so groß ist wie der von dem Stator 24 umschlossene Kanal. Außerdem entsteht am Umfang des Nabenteils 30 die Ringnut 40 (Fig. 2), in die der Ring 38 des Pumpengehäuses 32 eingeschnappt wird.

Damit das Lager 27, bei dem es sich um ein Wälzlager handelt, beim Injizieren des Polymermaterials nicht ausgespritzt wird, wird das Lager mit einem entfernbaren Material, wie Wachs oder Silikonfett, gefüllt, das später ausläuft oder als Lagerschmierstoff dient. Außerdem wird mit einem entsprechenden Füllmaterial ein Freiraum 66 (Fig. 2) offengehalten, um den freien Lauf des Lagers 27 zu gewährleisten.

Zu den Komponenten des Stators 24, die mit dem Polymermaterial 63 vergossen werden, gehört auch der Sensor 68 (Fig. 4), bei dem es sich hier um einen Drucksensor handelt. Mit einem Stopfen 69 wird das Druckfenster des Sensors 68 freigehalten. Dieser Stopfen 69 wird anschließend entfernt.

Die Spritzgußform enthält ferner Hohlräume für radiale Rippen 70 (Fig. 2), die an der Schräge des Nabenteils 30 vorgesehen sind. Diese Rippen dienen zur Abstützung der Stege 39 des Pumpengehäuses 32 und sie sind in Strömungsrichtung schräggestellt. Die Rippen 70 bewirken, daß das Pumpengehäuse 32 trotz der geringen Wandstärke der Stege 39 seine axiale Ausrichtung exakt beibehält.

Nach dem Aushärten des Polymermaterials 63 werden die Formhälften auseinandergenommen und das Motorgehäuse 20 wird zusammen mit dem darin enthaltenen Stator 24 von dem Dorn 60 abgezogen. Dann wird durch die Öffnung 31 der Rotor 26 eingeführt, wobei das Ende 25a der Welle 25 in das Lager 27 eindringt. An dem gegenüberliegenden Ende 25b der Welle 25 befindet sich das Lager 33, das zugleich als Dichtung wirkt. Beim Einschieben des Stators wird das auf der Welle sitzende Lager 33 in die Öffnung 31 des Motorgehäuses 20 eingepaßt. Dadurch wird das Motorgehäuse am distalen Ende abdichtend verschlossen. Auf dem aus der Dichtung 33 herausragenden Ende der Welle 25 sitzt das vorher bereits montierte Flügelrad 34. Am Schluß wird das Pumpengehäuse 32 mit seinem an den Stegen 39 befestigten Ring 38 in die Ringnut 40 des Motorgehäuses 20 eingeschnappt.

Der Durchmesser des Dornes 60 ist um etwa 2/10 mm größer als derjenige des Rotors 26, so daß zwischen Rotor und Stator ein Luftspalt von nur 1/10 mm entsteht. Ein so geringer Luftspalt ist mit dem beschriebenen Herstellungs- und Montageverfahren ohne Schwierigkeiten zu erzielen.

## Patentansprüche

1. Verfahren zur Herstellung eines Mikromotors (21) einer intrakardial oder intravasal zu betreibenden Blutpumpe (10), die ein in einem Pumpengehäuse (32) enthaltenes Flügelrad (34) aufweist, wobei der Mikromotor ein Motorgehäuse aufweist, das starr mit dem Pumpengehäuse (32) verbunden ist, mit den folgenden Schritten:
- Aufschieben von Statorkomponenten (24a, 24b) mit umfangsmäßig verteilt angeordneten Wicklungen (24a) auf einen starren Dorn (60),
- Einbringen des Dornes (60) mit den Statorkomponenten (24a, 24b) in eine Spritzgussform (50),
- Injizieren eines Polymermaterials (63) in einen Formhohlraum (53) der Spritzgussform (60) zur Bildung des Motorgehäuses (20), wobei der Formhohlraum (53) einen Stufenabsatz (55) zur Bildung einer Muffe (64) zum Aufschieben eines Katheters (24) enthält, wobei durch die Muffe Kabel (23) zur Stromversorgung und Steuerung des Motors verlaufen,
- Entfernen der Spritzgussform (50) und des Dornes (60) von dem Motorgehäuse (20) nach Aushärten des Polymermaterials,
- Einschieben eines Rotors (26) in das Motorgehäuse (20), wobei der Außendurchmesser des Rotors (26) etwa ²/₁₀ mm kleiner ist als derjenige des Dornes (60), wobei der Rotor (26) eine Motorwelle (25) aufweist, die außerhalb des Motorgehäuses (20) das Flügelrad (34) trägt.

2. Verfahren nach Anspruch 1, ferner mit dem Schritt des Aufschiebens eines Lagers (27) auf den Dorn (60) vor dem Einbringen des Domes (60) in die Spritzgussform (50).

3. Verfahren nach Anspruch 2, ferner mit dem Schritt des Füllens des Lagers (27) mit einem entfernbaren Material zur Vermeidung des Ausspritzens des Lagers (27) mit Polymermaterial.

4. Verfahren nach Anspruch 3, wobei das entfernbare Material Wachs oder eine ähnliche leicht schmelzbare oder durch Lösungsmittel entfernbare Substanz aufweist.

5. Verfahren nach einem der Ansprüche 1-4, wobei der Dorn (60) von einem Ende der Spritzgussform her in die Spritzgussform (50) eingebracht wird, während er am anderen Ende der Spritzgussform (50) in einem Kanal (54) der Spritzgussform (50) abgestützt wird und an dem anderen Ende eine Stirnwand (22) an das Motorgehäuse (20) angeformt wird.

6. Verfahren nach einem der Ansprüche 1-5, wobei außen an den Statorkomponenten (24a,24b) mindestens ein Sensor (68) befestigt wird, der mit Polymermaterial (63) umspritzt wird.

7. Verfahren nach Anspruch 6, wobei der Sensor (68) teilweise mit einer Abdeckung (69) bedeckt wird, welche nach dem Umspritzen wieder entfernt wird.

8. Verfahren nach einem der Ansprüche 1-7, wobei das Motorgehäuse (20) mit einer dem Querschnitt des einzusetzenden Rotors (26) entsprechenden Öffnung (31) hergestellt wird, durch die der Rotor (26) eingeschoben wird, auf dessen Welle (25) ein die Öffnung (31) verschließendes Lager (33) sitzt.

9. Verfahren nach einem der Ansprüche 1-8, wobei an das eine Ende des Motorgehäuses (20) radiale Rippen (70) angespritzt werden, die als Stützen für die Stege (39) eines Pumpengehäuses (32) benutzt werden.

10. Verfahren nach einem der Ansprüche 1-9, **dadurch gekennzeichnet, dass** Leistungsleitungen (23) und/oder Informationsleitungen (23a) mit dem Polymermaterial (63) umspritzt werden.

## Claims

1. A method for manufacturing a micro motor (21) of a intracardially or intravascularly operated blood pump (10) comprising an impeller (34) contained in a pump housing (32), said micro motor having a motor housing rigidly connected with the pump housing (32), the method comprising the following steps:
- slipping stator parts (24a, 24b) with windings (24a) distributed over the circumference onto a rigid mandrel (60),
- placing the mandrel (60) with the stator parts (24a, 24b) into an injection mold (50),
- injecting a polymer material (63) into a mold cavity (53) of the injection mold (60) for forming the motor housing (20), said mold cavity (53) comprising a step (55) for forming a sleeve (64) for slipping a catheter (24) thereon, the wires (23) for supplying current to the motor and fro controlling the same running through said sleeve,
- removing the injection mold (50) and the mandrel (60) from the motor housing (20) after the curing of the polymer material,
- inserting a rotor (26) into the motor housing (20), the outer diameter of the rotor (26) being about 2/10 mm smaller than that of the mandrel (60), the rotor (26) comprising a motor shaft (25) which supports the impeller (34) outside the motor housing (20).

2. The method of claim 1, further comprising the step of slipping a bearing (27) onto the mandrel (60) before placing the mandrel (60) into the injection mold (50).

3. The method of claim 2, further comprising the step of filling the bearing (27) with a removable material to avoid the filling of the bearing (27) with polymer material.

4. The method of claim 3, wherein the removable material comprises wax or a similar substance easy to melt or to remove with a solvent.

5. The method of one of claims 1 - 4, wherein the mandrel (60) is inserted into the injection mold (50) from one end, while the other end is supported in a channel (54) of the injection mold (50) and an end wall (22) is formed to the motor housing (20) at the other end.

6. The method of one of claims 1-5, wherein the outside of the stator parts (24a, 24b) is provided with at least one sensor encapsulated with polymer material (63).

7. The method of claim 6, wherein the sensor (68) is partly covered with a cover (69) which is removed after encapsulation.

8. The method of one of claims 1-7, wherein the motor housing (20) is manufactured with an opening (31) corresponding to the diameter of the rotor (26) to be installed, through which opening the rotor (26) is inserted, the shaft (25) of the rotor being provided with a bearing (33) closing the opening (31).

9. The method of one of claims 1-8, wherein radial ribs (70) are formed by injection molding on one end of the motor housing (20), the ribs being used as supports for the struts (39) of a pump housing (32).

10. The method of one of claims 1 - 9, **characterized in that** power lines (23) and/or information lines (23a) are encapsulated with the polymer material (63).

## Revendications

1. Procédé pour fabriquer un micromoteur (21) d'une pompe à sang (10) appelée à être exploitée par voie intracardiaque ou intravasculaire, qui présente une roue à ailettes (34) contenue dans un boîtier de pompe (32), le micromoteur présentant un boîtier de moteur, qui est réuni rigidement au boîtier de pompe (32), procédé comprenant les étapes suivantes :
- enfilage sur un mandrin rigide (60), de composants statoriques (24a, 24b) comprenant des enroulements (24a) disposés suivant une répartition circonférentielle,
- introduction du mandrin (60) avec les composants statoriques (24a, 24b), dans un moule d'injection (50),
- injection d'une matière polymère (63) dans une cavité de moulage (53) du moule d'injection (60) pour la formation du boîtier de moteur (20), la cavité de moulage (53) comportant un appendice à gradin (55) pour la formation d'un manchon (64) destiné à l'enfilage d'un cathéter (24), des câbles (23) passant à travers le manchon pour l'alimentation en courant et la commande du moteur,
- enlèvement du moule d'injection (50) et du mandrin (60) du boîtier de moteur (20), après durcissement de la matière polymère,
- insertion d'un rotor (26) dans le boîtier de moteur (20), le diamètre extérieur du rotor (26) étant plus petit d'environ 2/10 mm que celui du mandrin (60), le rotor (26) présentant un arbre de moteur (25), qui porte la roue à ailettes (34) à l'extérieur du boîtier de moteur (20).

2. Procédé selon la revendication 1, comprenant en outre l'étape d'enfilage d'un palier (27) sur le mandrin (60) avant l'introduction du mandrin (60) dans le moule d'injection (50).

3. Procédé selon la revendication 2, comprenant en outre l'étape de remplissage du palier (27) avec un matériau éliminable, afin d'éviter que le palier (27) soit souillé avec de la matière polymère injectée.

4. Procédé selon la revendication 3, dans lequel le matériau éliminable comprend de la cire ou une substance similaire, facilement fusible ou pouvant être éliminée facilement au moyen d'un solvant.

5. Procédé selon l'une des revendications 1 à 4, dans lequel le mandrin (60) est introduit dans le moule d'injection (50) par une extrémité du moule d'injection, tandis qu'à l'autre extrémité du moule d'injection (50), il est placé en appui dans un canal (54) du moule d'injection (50), et une paroi frontale (22) est formée sur le boîtier de moteur (20) à l'autre extrémité.

6. Procédé selon l'une des revendications 1 à 5, dans lequel sur les composants statoriques (24a, 24b) est fixé, du côté extérieur, au moins un capteur (68), qui est enveloppé de matière polymère (63) par injection.

7. Procédé selon la revendication 6, dans lequel le capteur (68) est couvert partiellement avec un élément de recouvrement (69), qui est à nouveau retiré après l'injection enveloppante.

8. Procédé selon l'une des revendications 1 à 7, dans lequel le boîtier de moteur (20) est fabriqué avec une ouverture (31) correspondant à la section transversale du rotor (26) à insérer, ouverture à travers laquelle est glissé le rotor (26), sur l'arbre (25) duquel est calé un palier (33) obturant l'ouverture (31).

9. Procédé selon l'une des revendications 1 à 8, dans lequel à l'une des extrémités du boîtier de moteur (20) sont rapportées, par injection, des nervures radiales (70), qui sont utilisées comme appuis pour les entretoises (39) d'un boîtier de pompe (32).

10. Procédé selon l'une des revendications 1 à 9, **caractérisé en ce que** des conducteurs de transport d'énergie (23) et/ou des conducteurs de transmission d'informations (23a) sont enveloppés, par injection, avec la matière polymère (63).
